(19) Europäisches Patentamt
European Patent Office
Office européen des brevets

(11) Veröffentlichungsnummer: **0 003 107**
**B1**

(12) # EUROPÄISCHE PATENTSCHRIFT

(45) Veröffentlichungstag der Patentschrift: **12.08.81**

(51) Int. Cl.³: **C 07 D 307/68**

(21) Anmeldenummer: **79100019.3**

(22) Anmeldetag: **04.01.79**

(54) **Verfahren zur Herstellung von Furancarbonsäureanilid.**

(30) Priorität: **05.01.78 DE 2800504**

(43) Veröffentlichungstag der Anmeldung: ***25.07.79 Patentblatt 79/15***

(45) Bekanntmachung des Hinweises auf die Patenterteilung: **12.08.81 Patentblatt 81/32**

(84) Benannte Vertragsstaaten: **BE CH DE FR GB IT NL SE**

(56) Entgegenhaltungen:
**DE-C-347 607**
**BULLETIN DE LA SOCIETE CHIMIQUE DE FRANCE, vol. 11, November 1971 Paris, FR.**
**E. BISAGNI et al. »Furannes et pyrroles disubstitués en 2,3. XI-Reaction du chloracétaldéhyde sur les $\beta$-cétoesters: formation des furannes ou pyrroles attendus et de diacyl-1,4 cyclohexadiènes-1,4« Seiten 4041—4047**

(73) Patentinhaber: **Consortium für elektrochemische Industrie GmbH, Zielstattstrasse 20, D-8000 München 70 (DE)**

(72) Erfinder: **Deinhammer, Wolfgang, Dr., Röntgenstrasse 32, D-8263 Burghausen (DE)**
Erfinder: **Kaufmann, Rudolf, Dr., Wackerstrasse 5, D-8263 Burghausen (DE)**
Erfinder: **Bräunling, Hermann, Dr., Dreyerstrasse 6, D-8000 München 21 (DE)**
Erfinder: **Häberle, Norman, Dr., Willibaldstrasse 51 a, D-8000 München 21 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 003 107 B1

BUNDESDRUCKEREI BERLIN

Verfahren zur Herstellung von Furancarbonsäureanilid

Die Erfindung betrifft die Darstellung von Furancarbonsäureanilid durch ein Zweistufenverfahren, wobei in der ersten Stufe Furancarbonsäureester und in einer zweiten Stufe Furancarbonsäureanilid hergestellt wird.

Nach Scott & Johnson, J. Am. Chem. Soc., 54, 2549 (1932) entstehen bei der Umsetzung von β-Dicarbonylverbindungen mit α-Halogencarbonylen in Gegenwart äquimolarer Mengen Pyridin Furanderivate mit Ausbeuten von 50 bis 60%. Auch in Gegenwart von Ammoniak, Soda, Natriumacetat, Triäthylamin, Natriumhydroxid oder Caliumhydroxid (E. Bisagni et al, Bull. Soc. Chem. France 1971 [11], 4041 ff,) werden keine besseren Ausbeuten berichtet. Ohne Angabe von Ausbeuten verläuft schließlich die Darstellung von 2-Methylfuran-3-carbonsäureäthylester gemäß der DE-OS 20 06 472, wobei allerdings 3 Mol Pyridin pro Mol β-Ketoester zum Einsatz gelangen. All diese Verfahren haben den Nachteil, daß entweder zu geringe Ausbeuten resultieren oder Pyridin in größeren Mengen mitverwendet werden muß. Die Abtrennung größerer Mengen Pyridin aus dem Reaktionsgemisch, sowie die Aufarbeitung pyridinhaltiger Abwässer ist in jedem Fall mit erheblichem technischem Aufwand verbunden.

Die Anilidierung von Furancarbonsäureestern durch direkte Umsetzung mit Anilin führt nicht zu technisch vertretbaren Ausbeuten und Reinheiten. Die Anilidierung vermittels Natriumanilid erfordert den technisch problematischen Umgang mit elementarem Natrium bzw. Natriumhydrid zur Herstellung des Natriumanilids.

Eine Verfahrensvariante Carbonsäureanilide aus Carbonsäureester und Halogenmagnesiumanilid zu erhalten, beinhaltet den Nachteil, im technischen Maßstab über eine Grignard-Reaktion gehen zu müssen. Zudem sind pro Mol Reaktionsprodukt 2 Mole der Grignard-Verbindung erforderlich. Aufgabe der Erfindung war es, ein Verfahren zur Herstellung von Furancarbonsäureaniliden zu finden, bei dem in der ersten und zweiten Stufe bessere Ausbeuten und Reinheiten resultieren. Die Aufarbeitung der Reaktionsprodukte soll gegenüber herkömmlichen Verfahren vereinfacht werden. Gegenstand der Erfindung ist ein Verfahren zur Herstellung von Furancarbonsäureanilid der allgemeinen Formel

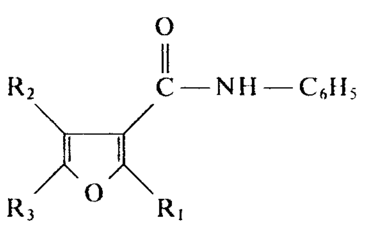

worin

$R_1$ eine Aryl- oder Alkylgruppe ist,

$R_2$ und $R_3$ eine Alkylgruppe oder Wasserstoff bedeuten,

durch Ringschlußreaktion von β-Ketoestern mit α-Halogenaldehyd bzw. α-Halogenketon, in Gegenwart zumindest stöchiometrischer Mengen halogenwasserstoffbindender Substanz zu Furancarbonsäureestern und anschließender Anilidierung, dadurch gekennzeichnet, daß die Ringschlußreaktion in Gegenwart der ein- bis zweifachen stöchiometrischen Menge, bezogen auf freigesetzte Halogenwasserstoffsäure, an einem Gemisch von Erdalkalicarbonat und Pyridin, wobei der Pyridinzusatz 0,1 bis 20 Mol-%, bezogen auf eingesetzte β-Dicarbonylverbindung beträgt, bei Temperaturen zwischen 50 und 100°C durchgeführt wird und der Furancarbonsäureester nach Abtrennung und Destillation unter Rühren mit Anilin in bis zu 20fachem molarem Überschuß in Gegenwart gleicher molarer Mengen, bezogen auf Furancarbonsäureester, von Magnesiumdianilid und/oder Aluminiumstrianilid in flüssiger Phase bzw. Suspension bei Temperaturen zwischen 20 und 180°C, bei Normaldruck, umgesetzt wird.

Bevorzugt bedeutet $R_1$ eine Methylgruppe sowie $R_2$ und $R_3$ Wasserstoff. Ebenso ist es bevorzugt, α-Chlor oder α-Brom-Aldehyde bzw. die entsprechenden Ketone einzusetzen.

Überraschend sind die guten Ausbeuten und die Reinheit des Produktes, die in beiden Stufen resultieren. Nicht zu erwarten war, daß bei Kenntnis der Vielzahl säurebindender Mittel gerade Erdalkalicarbonate die gewünschte Verbesserung der Ausbeute erbringen würden. Der gute Reaktionsablauf der ersten Stufe kann durch den Zusatz geringer Mengen Pyridin noch gesteigert werden. Es wurde aber gefunden, daß bereits katalystische Mengen zwischen 0,1 und 20 Mol-%, vorzugsweise 0,5 bis 5 Mol-%, Pyridin, bezogen auf die eingesetzten β-Dicarbonylverbindungen die Reaktion ausreichend beschleunigen. Bei Kenntnis der Veröffentlichung von Lazier und Atkins, J. Am. Chem. Soc. 46, 741 bis 743, mußte der Fachmann befürchten, daß während der Umsetzung der zweiten Reaktionsstufe das entstehende Magnesium- bzw. Aluminiumalkoholat ein schwer zu entfernendes, alkyliertes Anilid erzeugen würde. Als halogenwasserstoffbindende Substanzen werden in der ersten Reaktionsstufe erfindungsgemäß Erdalkalicarbonate eingesetzt. Beispielsweise geeignet ist Calciumcarbonat, sowohl in Form von gefälltem Calciumcarbonat als auch in natürlich vorkommenden Formen des Calciumcarbonats, wie z. B. Kalkstein oder Kreide. Vorzugsweise eignet sich natürlich vorkommender Dolomit aus Mischkristallisat von Calcium und Magnesiumcarbonat. Zweckmäßigerweise wird das Erdalkalicarbonat in gemahlener Form eingesetzt. Hierfür geeignete Teilchengrößen liegen zwischen 1 bis 200 μ. Die Erdalkalicarbonate werden

im allgemeinen in stöchiometrischen Mengen, bezogen auf den freigesetzten Halogenwasserstoff, eingesetzt. Unter Umständen kann es notwendig sein, geringfügig größere Erdalkalicarbonatmengen, insbesondere bei der Verwendung von Dolomit, einzusetzen. Ein Zusatz von Pyridin in Mengen von 0,1 bis 20 Mol-%, vorzugsweise 0,5 bis 5 Mol-%, beschleunigt die Reaktion erheblich. Auf den Einsatz molarer oder gar mehrfach molarer Mengen Pyridin kann verzichtet werden. Nach dem erfindungsgemäßen Verfahren ist es im Gegensatz zu bekannten Verfahren nicht notwendig, zusätzlich organische Verdünnungsmittel, Wasser oder verdünnte wäßrige Lösungen von halogenwasserstoffbindenden Substanzen einzusetzen. Daraus ergibt sich eine nicht unerhebliche Verringerung des Reaktionsvolumens gegenüber herkömmlicher Verfahrensweise. Die aus den Erdalkalicarbonaten sich im Verlauf der Reaktion bildenden Erdalkalihalogenide sind gut wasserlöslich, so daß sie mit der wäßrigen Phase aus während der Reaktion gebildetem Wasser bzw. nach der Reaktion zugesetztem Wasser entfernt werden können.

Die Reaktionstemperatur liegt im allgemeinen zwischen 50 und 100° C.

Die Darstellung von Durancarbonsäureester erfolgt am günstigsten bei Reaktionstemperaturen zwischen 65 und 80° C. Die Aufarbeitung des Furancarbonsäureesters erfolgt in üblicher Weise, z. B. durch Vakuumdestillation.

Das erfindungsgemäße Verfahren liefert höhere Ausbeuten als die bekannten Verfahren. Auf eine Rückgewinnung teuerer Stickstoffbasen kann verzichtet werden.

Der zweite Reaktionsschritt, die Umsetzung von Furancarbonsäureester mit Magnesiumdi- und/oder Aluminiumtrianilid, kann in bis zu 20fachem molarem Überschuß von Anilin, bezogen auf Carbonsäureester, bei Normaldruck und ggf. in Gegenwart eines inerten organischen Verdünnungsmittels durchgeführt werden.

Die Herstellung der erfindungsgemäß eingesetzten Anilide erfolgt durch Erhitzen einer Mischung von Metall und Anilin. Dabei wird das Metall in Form von Spänen eingesetzt, die beispielsweise in Gegenwart geringer Mengen eines Quecksilbersalzes aktiviert wurden. Bei Anilinüberschuß fällt das Metallanilid in Lösung bzw. Suspension an.

In diese Lösung bzw. Suspension von Magnesium- und/oder Aluminiumanilid wird unter Rühren der Carbonsäureester eingebracht. Wegen der auftretenden Reaktionswärme ist es mitunter notwendig zu kühlen. Anschließend an die Hauptreaktion ist es zweckmäßig, die Reaktionsmischung bei der Reaktionstemperatur 10 Minuten bis 24 Stunden zu belassen. Als Reaktionstemperatur der Umsetzung, die bei Normaldruck durchgeführt wird, sei 20 bis 180° C genannt, vorzugsweise wird bei 50 bis 140° C gearbeitet.

Die Zugabemenge des Carbonsäureesters ist äquimolar, bezogen auf Metallanilid. Natürlich kann als Verdünnungsmittel dem Reaktionsgemisch ein inerter Kohlenwasserstoff, wie beispielsweise Benzol, Toluol, Xylol, Cyclohexan oder Äther, wie Diäthyläther, Diisopropyläther, Dibutyläther, zugegeben werden. Vorzugsweise wird die Reaktion jedoch ausschließlich im überschüssigen Anilin der Metallanilidbildung durchgeführt. Die Abtrennung des überschüssigen Anilins nach der Reaktion bereitet keine Schwierigkeiten. Vorzugsweise wird ein Großteil des Anilins wegen seines, gegenüber dem Furancarbonsäureanilid stets niedrigeren Siedepunktes abdestilliert, vorzugsweise bei vermindertem Druck.

Das verbleibende Gemisch kann mit Wasser aufgearbeitet werden, wobei sich Alkohol, Carbonsäureanilid und Metallhydroxid bildet. Danach wird mit Mineralsäure, vorzugsweise Salzsäure oder Schwefelsäure, angesäuert und das in wäßrigem Medium unlösliche Furancarbonsäureanilid abfiltriert. Eine Aufarbeitungsvariante besteht darin, das Anilid in organischen, mit Wasser unter Phasentrennung mischbaren Lösungsmitteln ggf. in der Wärme aufzulösen, die Lösung mit Wasser zu vermischen, mit Mineralsäure anzusäuern und nach Trennung der wäßrigen von der organischen Phase die organische Phase aufzuarbeiten. Der verbleibende Rückstand kann umkristallisiert werden. Meist ist die Reinheit des Produktes jedoch so hoch, daß das Produkt zugleich als Handelsprodukt konfektioniert werden kann.

## Beispiel 1

### Darstellung von 2-Methylfuran-3-carbonsäuremethylester

In einem Rührkessel werden 58,055 kg (500 Mol) Acetessigsäuremethylester vorgelegt und 22,5 kg (487,5 Val) gemahlener Dolomit mit einer Korngröße um 30 a eingerührt. Nach Zugabe von 0,988 kg (12,5 Mol) Pyridin wird auf 70° C erwärmt und dann 87,2 kg (500 Mol) 45%ige wäßrige Chloracetaldehydlösung innerhalb etwa 1 Stunde zudosiert. Durch Kühlen wird die Reaktion bei 70° C gehalten. Etwa 4 Stunden nach Reaktionsbeginn muß schwach beheizt werden, nach 6 bis 7 Stunden wird die Temperatur auf 75 bis 80° C erhöht, daraufhin die Heizung abgestellt und noch einige Zeit bei fallender Temperatur weitergerührt. Das auf Raumtemperatur abgekühlte Reaktionsgemisch wird portionsweise mit insgesamt 10 l konzentrierter Salzsäure versetzt. Nach Phasentrennung wird die wäßrige Unterschicht abgetrennt, die organische Schicht mit 76 l Wasser gewaschen. Zur besseren Phasentrennung können ca. 2 kg Kochsalz zugegeben werden. Die organische Phase wird abgetrennt und bei vermindertem Druck destilliert. Die Ausbeute an 2-Methylfuran-3-carbonsäuremethylester beträgt 53,6 kg, das sind 76,6% der Theorie.

Beispiel 2
Darstellung von 2-Methyl-3-furancarboxanilid

18 g Aluminiumgranalien wurden mit einer 2%igen wäßrigen Quecksilber-I-Chloridlösung angeätzt, dann mit Wasser, Methanol, Toluol und Anilin gewaschen und schließlich mit 700 ml Anilin 10 Stunden unter Rückfluß gekocht, wobei Wasserstoffentwicklung auftrat. Dann wurde bei 110° C innerhalb von 10 Minuten 122 g 2-Methyl-3-furancarbonsäuremethylester unter Rühren eingebracht und 2 Stunden bei einer Temperatur von 110 bis 120° C weitergerührt. Die Lösung wurde dann noch heiß vom nicht umgesetzten Aluminium (2,3 g) abdekantiert, in Wasser gegossen, mit 20%iger wäßriger Salzsäure angesäuert und filtriert. Der neutral gewaschene und getrocknete Niederschlag von 2-Methyl-3-furancarboxanilid wog 172 g, das sind 94% der Theorie.

Beispiel 3
2-Methyl-3-furancarboxanilid

25 g Magnesiumspäne und 600 ml Anilin wurden 7 Stunden unter Ausschluß von Luftsauerstoff unter Rückfluß erhitzt, wobei Wasserstoffentwicklung auftrat, dann auf 120° abgekühlt und 140 g 2-Methyl-furan-3-carbonsäureäthylester innerhalb von 20 Minuten unter Rühren zugetropft. Nach 2stündigem Rühren bei 110 – 120° wurde das überschüssige Anilin im Vakuum abdestilliert. Die verbleibende Schmelze wurde in 400 ml Toluol bei 80° gelöst. Dann wurde bei 60 – 80° 100 ml Wasser und anschließend 350 ml 30%ige Salzsäure zugegeben. Die untere wäßrige Schicht wurde abgetrennt und kann zur Gewinnung des als Hydrochlorid gelösten Anilins durch Alkalischmachen weiterverarbeitet werden.

Die toluolische Lösung wurde noch zweimal bei 60 – 80° mit 5%iger Salzsäure, dann zweimal mit Wasser geschüttelt und ergab nach dem Abdampfen des Toluols 196 g 2-Methyl-3-furancarboxanilid, Reinheit 98%ig.

**Patentansprüche**

1. Verfahren zur Herstellung von Furancarbonsäureanilid der allgemeinen Formel

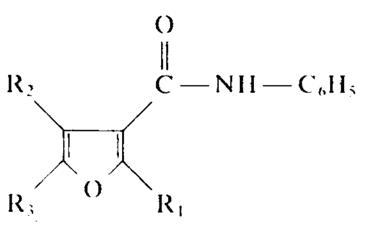

worin

$R_1$ eine Aryl- oder Alkylgruppe ist,
$R_2$ und $R_3$ eine Alkylgruppe oder Wasserstoff bedeuten,

durch Ringschlußreaktion von $\beta$-Ketoestern mit $\alpha$-Halogenaldehyd bzw. $\alpha$-Halogenketon, in Gegenwart zumindest stöchiometrischer Mengen halogenwasserstoffbindender Substanz zu Furancarbonsäureestern und anschließender Anilidierung, dadurch gekennzeichnet, daß die Ringschlußreaktion in Gegenwart der ein- bis zweifachen stöchiometrischen Menge, bezogen auf freigesetzte Halogenwasserstoffsäure, an einem Gemisch von Erdalkalicarbonat und Pyridin, wobei der Pyridinzusatz 0,1 bis 20 Mol-%, bezogen auf eingesetzte $\beta$-Dicarbonylverbindung beträgt, bei Temperaturen zwischen 50 und 100° C durchgeführt wird und der Furancarbonsäureester nach Abtrennung und Destillation unter Rühren mit Anilin in bis zu 20fachem molarem Überschuß in Gegenwart gleicher molarer Mengen, bezogen auf Furancarbonsäureester, von Magnesiumdianilid und/oder Aluminiumtrianilid in flüssiger Phase bzw. Suspension bei Temperaturen zwischen 20 und 180° C, bei Normaldruck, umgesetzt wird.

2. Verfahren nach Anspruch 1, dadurch gekennzeichnet, daß $R_1$ eine Methylgruppe ist.

3. Verfahren nach den Ansprüchen 1 und 2, dadurch gekennzeichnet, daß $R_2$ und $R_3$ Wasserstoff bedeutet.

4. Verfahren nach den Ansprüchen 1, 2 und 3, dadurch gekennzeichnet, daß unter Halogen Chlor oder Brom verstanden wird.

5. Verfahren nach den Ansprüchen 1, 2, 3 und 4, dadurch gekennzeichnet, daß die Ringschlußreaktion bei Temperaturen von 65 bis 80° C durchgeführt wird.

6. Verfahren nach den Ansprüchen 1, 2, 3, 4 und 5, dadurch gekennzeichnet, daß die Anilidierung bei Temperaturen von 50 bis 140° C durchgeführt wird.

**Claims**

1. Process for the manufacture of a furan carboxylic acid anilide of the general formula

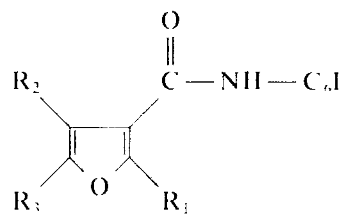

in which

$R_1$ is aryl group or an alkyl group, and
$R_2$ and $R_3$ denote an alkyl group or a hydrogen atom,

by a ring-closure reaction of $\beta$-ketoesters with $\alpha$-haloaldehydes or $\alpha$-haloketones, in the presence of at least stoichiometric amounts of hydrogen halide-binding substances to form furan carboxylic acid esters and by subsequent

anilidation, characterised in that the ring-closure reaction is carried out in the presence of one or two times the stoichiometric amount, based on the hydrohalic acid liberated, of a mixture of an alkaline earth metal carbonate and pyridine, the pyridine addition being from 0.1 to 20 mole % based on the $\beta$-dicarbonyl compound used, at temperatures of between 50 and 100° C and, after separation and distillation, the furancarboxylic acid ester is reacted, while being stirred, with aniline in up to 20 times molar excess, in the presence of equimolar amounts, based on the furancarboxylic acid ester, of magnesium dianilide and/or aluminium trianilide in the liquid phase or in suspension, at temperatures of between 20 and 180° C, under normal pressure.

2. Process according to claim 1, characterised in that $R_1$ is a methyl group.

3. Process according to claims 1 and 2, characterised in that $R_2$ and $R_3$ denote hydrogen.

4. Process according to claims 1, 2 and 3, characterised in that halogen is understood as being chlorine or bromine.

5. Process according to claims 1, 2, 3 and 4, characterised in that the ring-closure reaction is carried out at temperatures of from 65 to 80° C.

6. Process according to claims 1, 2, 3, 4 and 5 characterised in that the anilidation is carried out at temperatures of from 50 to 140° C.

**Revendications**

1. Procédé de préparation d'un anilide d'acide furanne-carboxylique répondant à la formule générale

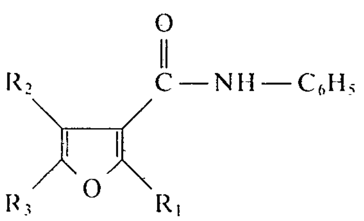

dans laquelle

$R_1$ représente un radical aryle ou alkyle et

$R_2$ et $R_3$ représentent chacun un radical alkyle ou un atome d'hydrogène,

par une réaction de cyclisation de $\beta$-céto-esters avec un $\alpha$-halogéno-aldéhyde ou une $\alpha$-halogéno-cétone, en présence de quantités au moins stoechiométriques d'un accepteur d'halogénure d'hydrogène, puis anilidation des esters d'acides furanne-carboxyliques formés, procédé caractérisé en ce qu'on effectue la réaction de cyclisation en présence d'une quantité représentant de 1 à 2 fois la quantité stoechiométrique, par rapport à l'halogénure d'hydrogène libéré, d'un mélange d'un carbonate de métal alcalino-terreux et de pyridine, la quantité de pyridine ajoutée étant comprise entre 0,1 et 20% en moles par rapport au composé $\beta$-dicarboxylique mis en jeu, à des températures comprises entre 50 et 100°C, et, après avoir séparé et distillé l'ester d'acide furanne-carboxylique, on le fait réagir, tout en agitant, avec l'aniline en un excès pouvant aller jusqu'à 20 fois la quantité molaire, en présence de quantités molaires égales, par rapport à l'ester d'acide furannecarboxylique, de magnésium-dianilide et/ou d'aluminium-trianilide, en phase liquide ou en suspension, à des températures comprises entre 20 et 180° C, sous la pression normale.

2. Procédé suivant la revendication 1, caractérisé en ce que $R_1$ représente un radical méthyle.

3. Procédé selon l'une des revendications 1 et 2, caractérisé en ce que $R_2$ et $R_3$ représentent chacun l'hydrogène.

4. Procédé selon l'une des revendications 1, 2 et 3, caractérisé en ce que l'halogène est le chlore ou le brome.

5. Procédé selon l'une des revendications 1, 2, 3 et 4, caractérisé en ce qu'on effectue la réaction de cyclisation à des températures comprises entre 65 et 80° C.

6. Procédé selon l'une des revendications 1, 2, 3, 4 et 5, caractérisé en ce que l'on effectue la conversion en anilide à des températures comprises entre 50 et 140° C.